# EUROPEAN PATENT SPECIFICATION

(11) **EP 4 404 911 B1**
(45) Date of publication and mention of the grant of the patent: **12.03.2025**
(21) Application number: 22789484.7
(22) Date of filing: 13.09.2022
(51) Int. Cl.: A61K 8/81, A61K 8/891, A61K 8/892, A61K 8/894, A61K 8/896, A61K 8/898, A61Q 5/02, A61Q 5/12

(54) **CONDITIONING SHAMPOO COMPOSITION**
KONDITIONIERSHAMPOO-ZUSAMMENSETZUNG
COMPOSITION DE SHAMPOOING CONDITIONNEUR

(30) Priority: 21.09.2021 EP 21198036
(43) Date of publication of application: 31.07.2024
(73) Proprietor: Unilever IP Holdings B.V., 3013 AL Rotterdam (NL); Unilever Global IP Limited, Wirral, Merseyside CH62 4ZD (GB)
(72) Inventor: EKANI NKODO, Axel Herve, 6708 WH Wageningen (NL); HAMER, Bethany Rebecca Louise, 6708 WH Wageningen (NL); MOGHADAM, Arash Mohajer, 6708 WH Wageningen (NL)
(74) Representative: Mathai, Neenu Grace
(86) International application number: PCT/EP2022/075362
(87) International publication number: WO 2023/046529

(56) References cited:
- WO-A1-2016/113316
- WO-A1-2017/153262
- WO-A2-2013/186720
- US-A1- 2003 091 523

## Description

### Field of the Invention

The present invention is in the field of rinse-off compositions; in particular, relates to shampoo compositions for improved hair conditioning and styling properties.

### Background of the Invention

The tactile texture of the human hair is one of the most important factors in designing shampoos, hair conditioners, and hair styling agents. Human hair with soft and breezy texture is desirable for many customers. Therefore, the hair's tactile texture and frictional properties are constantly evaluated after treatment with hair-care products.

Various physical phenomena affect the tactile texture of human hair. For example, hair friction increases with activities such as bleaching damages the hair causing hindrance while combing and leading to unhealthy looking hair. Use of conditioning agents in hair compositions makes hair easier to comb when wet and more manageable when dry as they are adsorbed onto the hair surface and induce a smooth feeling. Commonly used conditioning agents include cationic surfactants and polymers, high melting point fatty compounds, low melting point oils, silicone compounds, and mixtures thereof. Silicones are well known and are one of the preferred conditioning agents. However, increasing silicone concentrations in the hair compositions to achieve better conditioning can contribute to undesirable, heavy and coated feel for users of the product.

There is a constant need to improve conditioning properties such as low friction and ease of combing for hair after drying, with a reduction in the heavy, greasy feel.

Pressure sensitive adhesives (PSAs) have been used in hair care compositions as described in WO2016/113316, WO2017/153262, US2003/091523, WO2013/186720, US5166276, EP412707 and EP412704. Pressure sensitive adhesives are known to provide hair style retention benefits.

It is an object of the present invention to provide a hair treatment composition which can provide improved hair conditioning and styling properties.

It is another object of the present invention to provide hair conditioning and styling properties from a single composition which is a shampoo composition.

It is yet another object of the present invention to provide a hair treatment composition which can improve low friction without increasing the conditionings agents for eg: silicones.

Surprisingly, it has been found that using specific pressure sensitive adhesives in combination with a silicone can improve hair conditioning and styling properties.

### Summary of the Invention

Accordingly, in a first aspect, the present invention provides a hair cleansing composition comprising: 1% to 50% by weight of one or more cleansing surfactants; 0.1% to 5% by weight of a pressure sensitive adhesive, said pressure sensitive adhesive being a random copolymer comprising an acrylic group having a side chain with at least 4 carbons; and a C₁-C₆ side chain acrylic; and 0.5% to 3% by weight of a silicone.

In a second aspect, the invention provides use of a pressure sensitive adhesive comprising an acrylic group having a side chain with at least 4 carbons; and a C₁-C₆ side chain acrylic in a shampoo composition comprising silicone to improve the reduction of dry friction on hair.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed Description of the Invention

In a first aspect, the present invention relates to a hair cleansing composition comprising a pressure sensitive adhesive and a silicone.

### Pressure sensitive adhesive

"Pressure sensitive adhesive" (PSA) materials are permanently tacky at room temperature and able to develop measurable adhesion to a surface simply upon contact or by the application of a light pressure.

Acrylic PSAs are random copolymers comprising an acrylic group having a side-chain with at least 4 carbons (eg n-butyl acrylate or 2-ethylhexyl acrylate) and having a low glass transition temperature Tg, a short side-chain acrylic such as methyl acrylate to adjust the Tg, and acrylic acid to improve adhesion and optimise elongational properties (i.e., their mechnical response to deformation in uniaxial extension). Small molecule additives such as tackifiers may be included, essentially to adjust the Tg and optimise dissipative properties but are not essential.

The pressure sensitive adhesive of the present invention are random copolymers comprising
i An acrylic group having a side chain with at least 4 carbons; and
ii A C₁-C₆ side chain acrylic.

Examples of an acrylic group having a side chain with at least 4 carbons include n-butyl acrylate and 2-ethylhexyl acrylate, n-hexyl acrylate, isooctyl acrylate and dodecyl acrylate.

Preferred acrylic group having a side chain of 4 or more carbon are butyl acrylate and 2-ethyl hexyl acrylate.

Examples of C₁-C₆ side chain acrylic include acrylic acid, methyl acrylate, methyl methacrylate, ethyl acrylate, butyl acrylate, methacrylic acid and butyl methacrylate.

Preferred C₁-C₆ side chain acrylic are selected from one or more of acrylic acid, methacrylic acid or butyl methacrylate.

Suitable water borne acrylic pressure sensitive adhesives include Dow Corning PA-0560, Dow Corning PA-0580, Dow Corning MG-0560, Dow Corning MG-0580, NACOR 38-088A ex National Starch and Chemical, Acudyne MD-5800 by Dow, Acudyne MD-5600 by Dow, Tackwhite NA 55 ex Ichemco srl, Tackwhite A 4 MED ex Ichemco srl, Acronal 80 D ex BASF AG, Acronal 85 D BASF AG, Acronal A220 exBASF AG, Acronal N 285 ex BASF AG, Acronal V 210 ex BASF AG and Acronal V212 ex BASF AG.

Particularly preferred acrylic pressure sensitive materials include Acudyne MD-5800 by Dow and Acudyne MD-5600 (RODERM^{™} MD 5600) by Dow, the most preferred being RODERM^{™} MD 5600.

The pressure sensitive adhesive of the present invention preferably has a dynamic storage (G') value of 10³ Pa to 10⁶ Pa.

The pressure sensitive adhesives of the present invention preferably have a dissipation (G") value of 10³ Pa to 10⁶ Pa.

The glass transition temperature of the pressure sensitive adhesive is preferably -100°C to 20°C, more preferably -80°C to 0°C and most preferably -60°C to -30°C.

The pressure sensitive adhesive is present in the cleansing composition in a concentration of 0.1% to 5%, preferably at least 0.5%, more preferably at least 1%, still more preferably at least 1.8% but typically not more than 4%, more preferably not more than 3%, even more preferably not more than 2% by weight of the total composition.

### Silicones

The silicones used in the present invention includes any of the silicones used or usable in hair treatment compositions as conditioning agents.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the silicone itself (not the emulsion or the final hair conditioning composition) is typically from 350 to 200,000,000 mm² sec⁻¹ at 25°C. Preferably the viscosity is at least 5,000 mm² sec⁻¹ at 25°C, more preferably at least 10,000 mm² sec⁻¹. Preferably the viscosity does not exceed 20,000,000 mm² sec⁻¹, more preferably 10,000,000 mm² sec⁻¹, most preferably 5,000, 000 mm² sec⁻¹.

Viscosities are generally provided by suppliers of silicones, either as measured or as deduced from their molecular weight.

Silicones used in the present invention may also include functionalised silicone. Suitable functionalised silicones include, amino-, carboxy-, betaine-, quaternary ammonium-, carbohydrate-, hydroxy-and alkoxy-substituted silicones. Preferably, the functionalised silicone contains multiple substitutions.

For the avoidance of doubt, as regards hydroxyl-substituted silicones, a polydimethylsiloxane merely having hydroxyl end groups (which have the CTFA designation dimethiconol) is not considered a functionalised silicone within the definition of the present invention. However, a polydimethylsiloxane having hydroxyl substitutions along the polymer chain is considered a functionalised silicone.

A preferred class of functionalised silicone for inclusion in compositions of the invention is amino functional silicone. By "amino functional silicones" is meant a silicone containing at least one primary, secondary or tertiary amine group, or a quaternary ammonium group.

Examples of suitable amino functional silicones include: polysiloxanes having the CTFA designation "amodimethicone".

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones). Suitable quaternary silicone polymers are described in EP-A- 0 530 974. A preferred quaternary silicone polymer is K3474, ex Goldschmidt.

Another preferred functional silicone is an alkoxy-substituted silicone. Such molecules are known as silicone copolyols and have one or more polyethyleneoxide or polypropyleneoxide groups bonded to the silicone polymer backbone, optionally through an alkyl linking group.

An example of a type of silicone copolyol useful in compositions of the invention has a molecular structure according to the formula depicted below:

Si(CH₃)₃[O-Si(CH₃)(A)]ₚ-[O-Si(CH₃)(B)]_{q}-O-Si(CH₃)₃

In this formula, A is an alkylene chain with from 1 to 22 carbon atoms, preferably 4 to 18, more preferably 10 to 16. B is a group with the structure : - (R)-(EO)ᵣ(PO)ₛ-OH wherein R is a linking group, preferably an alkylene group with 1 to 3 carbon atoms.

Preferably R is- (CH₂)₂-. The mean values of r and s are 5 or more, preferably 10 or more, more preferably 15 or more. It is preferred if the mean values of r and s are 100 or less. In the formula, the value of p is suitably 10 or more, preferably 20 or more, more preferably 50 or more and most preferably 100 or more. The value of q is suitably from 1 to 20 wherein the ratio p/q is preferably 10 or more, more preferably 20 or more. The value of p + q is a number from 11 to 500, preferably from 50 to 300.

Suitable silicone copolyols have an HLB of 10 or less, preferably 7 or less, more preferably 4 or less. A suitable silicone copolyol material is DC5200, known as Lauryl PEG/PPG-18/18 methicone (INCI name), available from Dow Corning.

It is preferred to use a combination of amino and non- functional silicones. In particular, when the silicone is a silicone oil blend, it is preferred if the silicone oil blend comprises
(i) from 50 to 95% by weight of the total weight of silicone oil of a polydimethylsiloxane gum having a molecular weight of at least 200,000 Daltons and
(ii) from 5 to 50% by weight of the total weight of silicone oil a second silicone which is an amino- functionalised polydimethylsiloxane having a molecular weight of less than 200,000 Daltons.

Suitable amino-functionalised silicones for such a blend are described in EP 455,185 (Helene Curtis).

An example of a commercially available amino-functionalised silicone useful in the composition of the invention is DC-8220 available from Dow Corning, which has a viscosity of 150 mm² s⁻¹ at 25°C and a mole percent amine functionality of 2. 0%.

The silicones may be added to the composition as a fluid and subsequently emulsified, but preferably are added as pre- formed emulsions for ease of processing.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The silicone used in the present invention include non-linear, preferably branched organopolysiloxane comprising monomer units of silsesquioxane having a formula (RSiO_{3/2})ₙ
where n = 1;
R is an alkyl group, preferably methyl, ethyl or propyl, more preferably methyl; and copolymer segments of polydialkylsiloxane, wherein the alkyl groups are preferably methyl, and the end groups are preferably -OH; the most preferred polydialkylsiloxane is dimethiconol.

Silicones used in the present invention may also include cyclomethicones.

The silicones may be present in the composition in a concentration of 0.1 to 3%, preferably at least 0.25%, more preferably at least 0.5%, still more preferably at least 0.75%, even more preferably at least 1% but typically not more than 2.9%, preferably not more than 2.5%, more preferably not more than 2%, still more preferably not more than 1.5% by weight of the composition.

The cleansing composition according to the invention comprises pressure sensitive adhesive and silicone in a ratio of 4:1 to 1:4.

### Shampoos

Shampoo compositions of the invention are generally aqueous, i.e. they have water or an aqueous solution or a lyotropic liquid crystalline phase as their major component.

Suitably, the shampoo composition will comprise from 50 to 98%, preferably from 60 to 92% water by weight based on the total weight of the composition.

Surfactants are compounds which have hydrophilic and hydrophobic portions that act to reduce the surface tension of the aqueous solutions they are dissolved in. Shampoo compositions according to the invention will generally comprise one or more cleansing surfactants, which are cosmetically acceptable and suitable for topical application to the hair. The cleansing surfactant may be chosen from anionic, non-ionic, amphoteric and zwitterionic compounds and mixtures thereof.

The total amount of cleansing surfactant in a shampoo composition for use in the invention is generally from 1 to 50%, preferably from 2 to 40%, more preferably from 4 to 25% by total weight surfactant based on the total weight of the composition.

Non-limiting examples cleansing surfactants include anionic cleansing surfactants include; alkyl sulphates, alkyl ether sulphates, alkaryl sulphonates, N-alkyl sarcosinates, alkyl phosphates, alkyl ether phosphates, acyl amino acid based surfactants, alkyl ether carboxylic acids, acyl taurates, acyl glutamates, alkyl glycinates and salts thereof, especially their sodium, magnesium, ammonium and mono-, di- and triethanolamine salts. The alkyl and acyl groups in the preceding list generally contain from 8 to 18, preferably from 10 to 16 carbon atoms and may be unsaturated. The alkyl ether sulphates, alkyl ether phosphates and alkyl ether carboxylic acids and salts thereof may contain from 1 to 20 ethylene oxide or propylene oxide units per molecule.

Further non-limiting examples of cleansing surfactants may include non-ionic cleansing surfactants including; aliphatic (C₈ - C₁₈) primary or secondary linear or branched chain alcohols with alkylene oxides, usually ethylene oxide and generally having from 6 to 30 ethylene oxide groups. Other representative cleansing surfactants include mono- or dialkyl alkanolamides (examples include coco mono-ethanolamide and coco monoisopropanolamide) and alkyl polyglycosides (APGs). Suitable alkyl polyglycosides for use in the invention are commercially available and include for example those materials identified as: Plantapon 1200 and Plantapon 2000 ex BASF. Other sugar-derived surfactants, which can be included in compositions for use in the invention include the C₁₀-C₁₈ N-alkyl (C_{I}-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

Additional non-limiting examples of cleansing surfactants may include amphoteric or zwitterionic cleansing surfactants including; alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms.

Typical cleansing surfactants for use in shampoo compositions for use in the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium cocoyl isethionate, sodium lauryl isethionate, lauryl ether carboxylic acid and sodium N-lauryl sarcosinate, sodium pareth sulphate, cocodimethyl sulphopropyl betaine, lauryl betaine, coco betaine, cocamidopropyl betaine, sodium cocoamphoacetate.

Preferred cleansing surfactants are sodium lauryl sulphate, sodium lauryl ether sulphate (n)EO, (where n is from 1 to 3), ammonium lauryl sulphate, ammonium lauryl ether sulphate(n)EO, (where n is from 1 to 3), sodium cocoyl isethionate and lauryl ether carboxylic acid, coco betaine, cocamidopropyl betaine, sodium cocoamphoacetate.

Mixtures of any of the foregoing anionic, non-ionic and amphoteric cleansing surfactants may also be suitable, preferably where the primary to secondary surfactant ratio is between 1:1 - 10:1, more preferably 2:1 - 9:1 and most preferably 3:1 - 8:1, based on the inclusion weight of the cleansing surfactant in the shampoo composition.

Optionally, a shampoo composition for use in the invention may contain further ingredients, (non-limiting examples of which are described below) to enhance performance and/or consumer acceptability.

Cationic polymers are preferred ingredients in a shampoo composition for use in the invention for enhancing conditioning performance.

Suitable cationic polymers may be homopolymers which are cationically substituted or may be formed from two or more types of monomers. The weight average (M_{w}) molecular weight of the polymers will generally be between 100 000 and 3 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions for use in the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581). Examples of such materials include the polymer LR and JR series from Dow, generally referred to in the industry (CTFA) as Polyquaternium 10.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

Cationic polymer will generally be present in a shampoo composition for use in the invention at levels of from 0.01 to 5%, preferably from 0.02 to 1%, more preferably from 0.05 to 0.8% by total weight of cationic polymer based on the total weight of the composition.

Unless otherwise indicated, ratios, percentages, parts, and the like, referred to herein, are by weight.

### Other Ingredients

The composition of the invention may contain further optional ingredients to enhance performance and/or consumer acceptability. Examples of such ingredients include fragrance, dyes and pigments. Each of these ingredients will be present in an amount effective to accomplish its purpose. Generally, these optional ingredients are included individually at an amount of up to 5% (by weight based on the total weight of the composition).

The composition of the invention is primarily intended for topical application to the hair and scalp.

Most preferably the composition of the invention is topically applied to the hair and then massaged into the hair and scalp. The composition is then rinsed off the hair and scalp with water prior to drying the hair.

In a second aspect the present invention relates to use of a pressure sensitive adhesive comprising an acrylic group having a side chain with at least 4 carbons; and a C₁-C₆ side chain acrylic in a shampoo composition comprising silicone to improve the reduction of dry friction on hair.

The invention will be further illustrated by the following, non-limiting Examples.

### Examples

### Example 1: Effect of shampoo compositions comprising PSA and silicone on dry friction

Rinse-off aqueous hair cleansing shampoo formulations comprising PSA and silicone were prepared and compared with shampoo formulations containing either silicone or PSA.

The prepared formulations were used on hair switches and friction was measured. Friction was measured using the texture analyser to obtain the dry friction value.

### Friction measurement methodology

All friction measurements were carried out under controlled conditions of 20 degrees and 50 % RH.

Friction was measured using a TA.XT2i Texture Analyser supplied by Stable Micro Systems, Surrey, UK. The friction probe was a stainless steel cylinder, which was coated with rubber material. The load on the friction contact was approximately 560 g. When in use, an area of contact between the friction probe and the hair of approximately 1.0 cm² was achieved.

The methodology used to assess the friction properties of hair treated with test conditioners was as follows:
A switch of hair was securely mounted onto the texture analyser, the hair fibres being aligned by combing before being secured in a flat configuration. The friction probe was placed onto the hair and moved along the hair at a speed of 10 mms⁻¹ to measure the friction between the probe and the hair.

The friction values reported below are of friction hysteresis in units of g.mm, obtained by integrating the total measured friction force over the total distance travelled by the probe, with and against cuticle. Two measurements were performed per switch (one up and one down the hair switch) and 5 switches per formulation were tested. The values reported are the mean values derived from these ten readings per treatment.

**Table 1a**

| Shampoo compositions | Ratio of PSA to Silicone | Mean Dry TA Friction (g.mm) |
|---|---|---|
| **14% SLES (Control)** | - | 57956 |
| **1% Silicone** | - | 20166 |
| **1% Silicone, 2% MD5600** | 2:1 | 15752 |
| **1% Silicone, 2% MD5800P** | 2:1 | 18502 |
| **2% MD5600** | - | 58519 |
| **2% MD5800P** | - | 59012 |

| | | |
|---|---|---|
| Silicone - Dowsil CE-7051 POE Emulsion silicone blend ex Dow MD5600- Acudyne MD-5600 (RODERM^{™} MD 5600) by Dow MD5800P - Acudyne MD-5800 by Dow | | |

The above table shows that shampoo compositions with both PSA and silicone exhibit improved friction reduction when compared to shampoo formulations containing either of silicone or PSA.

The below table shows shampoo composition comprising PSA and silicone in various ratios.

**Table 1b**

| Shampoo compositions | Ratio of PSA to Silicone | Mean Dry TA Friction (g.mm) | SD |
|---|---|---|---|
| **Shampoo (Control)** | - | 56659.942 | 2510.214468 |
| **1.5% PSA, 0.5% Silicone** | 3:1 | 24657.4 | 3068.870624 |
| **2% PSA, 0.5% Silicone** | 4:1 | 28918.076 | 1880.417898 |
| **1% PSA, 0.75% Silicone** | 1.3:1 | 20672.58 | 1331.500897 |
| **1.5% PSA, 0.75% Silicone** | 2:1 | 28714.202 | 7088.61183 |
| **2% PSA, 0.75% Silicone** | 2.6:1 | 26812.438 | 4307.932985 |
| **0.5% PSA, 1% Silicone** | 1:2 | 20885.84 | 3675.649341 |
| **1% PSA, 1% Silicone** | 1:1 | 23719.776 | 6713.057986 |
| **1.5% PSA, 1% Silicone** | 1.5:1 | 23153.63 | 4182.945674 |
| **0.5% PSA, 1.5% Silicone** | 1:3 | 17073.152 | 2046.546241 |
| **0.5% PSA, 2% Silicone** | 1:4 | 18729.348 | 5567.993857 |
| **1% PSA, 0% Silicone** | - | 55717.72 | 3063.80401 |
| **1.5% PSA, 0% Silicone** | - | 56066.218 | 3881.008004 |
| | | | |

| | | | |
|---|---|---|---|
| Silicone - Dowsil CE-7051 POE Emulsion silicone blend ex Dow | | | |

## Claims

1. A hair cleansing composition comprising:
a 1% to 50% by weight of one or more cleansing surfactants;
b 0.1% to 5% by weight of a pressure sensitive adhesive, said pressure sensitive adhesive being a random copolymer comprising
i An acrylic group having a side chain with at least 4 carbons; and
ii A C₁-C₆ side chain acrylic; and
c 0.5% to 3% by weight of a silicone.

2. A hair cleansing composition according to claim 1 wherein the composition comprises pressure sensitive adhesive and silicone in a ratio of 4:1 to 1:4.

3. A hair cleansing composition according to claim 1 or 2 wherein the surfactant is selected from anionic, non-ionic, amphoteric, zwitterionic compounds or mixtures thereof.

4. A hair cleansing composition according to anyone of the preceding claims, wherein the acrylic group is butyl acrylate or 2-ethyl hexyl acrylate.

5. A hair cleansing composition according to anyone of the preceding claims, wherein the C₁-C₆ side chain acrylic is selected from one or more of acrylic acid, methacrylic acid or butyl methacrylate.

6. A hair cleansing composition according to anyone of the preceding claims wherein the composition comprises pressure sensitive adhesive and silicone in a ratio of 3:1 to 1:3.

7. A hair cleansing composition according to any one of the preceding claims wherein composition comprises pressure sensitive adhesive and silicone in a ratio of 2:1 to 1:2.

8. A hair cleansing composition according to anyone of the preceding claims wherein composition comprises 50 to 98% by weight of water.

9. Use of a pressure sensitive adhesive comprising an acrylic group having a side chain with at least 4 carbons; and a C₁-C₆ side chain acrylic in a shampoo composition comprising silicone to improve the reduction of dry friction on hair.

## Patentansprüche

1. Haarreinigungszusammensetzung, umfassend:
a 1 bis 50 Gewichts-% eines oder mehrerer Reinigungstenside;
b 0,1 bis 5 Gewichts-% eines druckempfindlichen Klebstoffs, wobei der druckempfindliche Klebstoff ein statistisches Copolymer ist, umfassend
i eine Acrylgruppe mit einer Seitenkette mit mindestens 4 Kohlenstoffatomen und
ii ein C₁-C₆-Seitenkettenacryl und
c 0,5 bis 3 Gewichts-% eines Silikons.

2. Haarreinigungszusammensetzung nach Anspruch 1, wobei die Zusammensetzung druckempfindlichen Klebstoff und Silikon in einem Verhältnis von 4:1 bis 1:4 umfasst.

3. Haarreinigungszusammensetzung nach Anspruch 1 oder 2, wobei das Tensid unter anionischen, nicht-ionischen, amphoteren und zwitterionischen Verbindungen oder Mischungen davon ausgewählt ist.

4. Haarreinigungszusammensetzung nach irgendeinem der vorhergehenden Ansprüche, wobei die Acrylgruppe Butylacrylat oder 2-Ethylhexylacrylat ist.

5. Haarreinigungszusammensetzung nach irgendeinem der vorhergehenden Ansprüche, wobei das C₁-C₆-Seitenkettenacryl aus einer oder mehreren der folgenden Substanzen ausgewählt ist: Acrylsäure, Methacrylsäure oder Butylmethacrylat.

6. Haarreinigungszusammensetzung nach irgendeinem der vorhergehenden Ansprüche, wobei die Zusammensetzung druckempfindlichen Klebstoff und Silikon in einem Verhältnis von 3:1 bis 1:3 umfasst.

7. Haarreinigungszusammensetzung nach irgendeinem der vorhergehenden Ansprüche, wobei die Zusammensetzung druckempfindlichen Klebstoff und Silikon in einem Verhältnis von 2:1 bis 1:2 umfasst.

8. Haarreinigungszusammensetzung nach irgendeinem der vorhergehenden Ansprüche, wobei die Zusammensetzung 50 bis 98 Gewichts-% Wasser umfasst.

9. Verwendung eines druckempfindlichen Klebstoffs, umfassend eine Acrylgruppe mit einer Seitenkette mit mindestens 4 Kohlenstoffatomen und ein C₁-C₆-Seitenkettenacryl in einer Shampoozusammensetzung, umfassend Silikon, um die Trockenreibung auf dem Haar zu verringern.

## Revendications

1. Composition nettoyante capillaire comprenant :
a. 1 % à 50 % en poids d'un ou plusieurs tensioactifs nettoyants ;
b. 0,1 % à 5 % en poids d'un adhésif sensible à la pression, ledit adhésif sensible à la pression étant un copolymère aléatoire comprenant
i. un groupe acrylique ayant une chaîne latérale avec au moins 4 carbones ; et
ii. un acrylique à chaîne latérale en C₁ à C₆ ; et
c. 0,5 % à 3 % en poids d'une silicone.

2. Composition nettoyante capillaire selon la revendication 1, laquelle composition comprend l'adhésif sensible à la pression et la silicone en un rapport de 4/1 à 1/4.

3. Composition nettoyante capillaire selon la revendication 1 ou 2, dans laquelle le tensioactif est choisi parmi les composés anioniques, non-ioniques, amphotères, zwittérioniques et leurs mélanges.

4. Composition nettoyante capillaire selon l'une quelconque des revendications précédentes, dans laquelle le groupe acrylique est l'acrylate de butyle ou l'acrylate de 2-éthylhexyle.

5. Composition nettoyante capillaire selon l'une quelconque des revendications précédentes, dans laquelle l'acrylique à chaîne latérale en C₁ à C₆ est un ou plusieurs choisis parmi l'acide acrylique, l'acide méthacrylique et le méthacrylate de butyle.

6. Composition nettoyante capillaire selon l'une quelconque des revendications précédentes, laquelle composition comprend l'adhésif sensible à la pression et la silicone en un rapport de 3/1 à 1/3.

7. Composition nettoyante capillaire selon l'une quelconque des revendications précédentes, laquelle composition comprend l'adhésif sensible à la pression et la silicone en un rapport de 2/1 à 1/2.

8. Composition nettoyante capillaire selon l'une quelconque des revendications précédentes, laquelle composition comprend 50 à 98 % en poids d'eau.

9. Utilisation d'un adhésif sensible à la pression comprenant un groupe acrylique ayant une chaîne latérale avec au moins 4 carbones ; et un acrylique à chaîne latérale en C₁ à C₆, dans une composition de shampooing comprenant de la silicone pour améliorer la réduction du frottement à sec sur les cheveux.
